# EUROPÄISCHE PATENTSCHRIFT

(11) **EP 3 064 274 B1**
(45) Veröffentlichungstag und Bekanntmachung des Hinweises auf die Patenterteilung: **16.08.2017**
(21) Anmeldenummer: 16158044.4
(22) Anmeldetag: 01.03.2016
(51) Int. Cl.: B01J 38/56, C07C 2/12, C07C 11/02, C07C 11/08, C07C 11/107, C07C 41/06, C07C 2/10, C07C 2/24

(54) **REGENERATION EINES IN DER ETHEN-OLIGOMERISIERUNG EINGESETZTEN, HETEROGENEN KATALYSATORS**
REGENERATION OF A HETEROGENEOUS CATALYST USED IN ETHENE OLIGOMERISATION
RÉGÉNÉRATION D'UN CATALYSEUR HÉTÉROGÈNE UTILISÉ DANS L'OLIGOMÉRISATION D'ÉTHÈNE

(30) Priorität: 03.03.2015 EP 15157358
(43) Veröffentlichungstag der Anmeldung: 07.09.2016
(73) Patentinhaber: Evonik Degussa GmbH, 45128 Essen (DE)
(72) Erfinder: STOCHNIOL, Guido, 45721 Haltern am See (DE); PEITZ, Stephan, 45739 Oer-Erkenschwick (DE); MASCHMEYER, Dietrich, 45657 Recklinghausen (DE); REEKER, Helene, 44227 Dortmund (DE)

(56) Entgegenhaltungen:
- WO-A1-2004/080591
- WO-A1-2006/022803
- WO-A1-2011/136765
- DE-A1-102005 026 213

## Beschreibung

Die Erfindung beschäftigt sich mit der Regeneration von heterogenen Katalysatoren, die in der Flüssigphasen- Oligomerisierung von Ethen eingesetzt werden.

Ethen (synonym: Ethylen) ist das einfachste Olefin (Alken). Seine Summenformel ist C₂H₄; es weist mithin zwei Kohlenstoffatome auf und wird auch als C₂-Olefin bezeichnet. Aufgrund seiner hohen Reaktivität stellt Ethen einen wichtigen Synthesebaustein der chemischen Industrie dar.

Die weitaus größte Menge des Ethens wird polymerisiert zu Polyethylen, einem der am weitesten verbreiteten Massenkunststoffe, der uns überall ständig begegnet, meist in Form von Verpackungs-Folie. Das Polyethylen ist ein Kettenpolymer, in dem das Ethen-Monomer [-CH₂-CH₂-] als Kettenglied vielfach wiederholt ist. Ein Polyethylen-Kettenpolymer weist daher eine sehr große Anzahl an Kohlenstoff-Atomen auf, weit mehr als 96.

Aus Ethen lassen sich aber auch andere Olefine mit vier, sechs oder acht Kohlenstoffatomen herstellen. Dies geschieht im Wege der Oligomerisierung. Bei der Ethen-Oligomerisierung reagiert Ethen im Wesentlichen mit sich selbst und bildet dabei Olefine mit mehr als zwei Kohlenstoffatomen, so genannte Oligomere. So können beispielsweise zwei Ethen-Moleküle zu einem Buten-Molekül reagieren, also einem Olefin mit vier Kohlenstoffatomen (kurz: C₄-Olefin). Man spricht bei dieser Reaktion auch von Dimerisierung, das Buten ist das Dimer des Ethens. Drei Ethen-Moleküle können gemeinsam ein Hexen (C₆-Olefin) bilden (Trimerisierung) und vier Ethen-Moleküle ein C₈-Olefin als Oligomer bilden (Tetramerisierung). Parallel können zwei zuvor gebildete Butene ein Octen bilden. In der Oligomerisierung laufen nämlich stets mehrere Reaktionen gleichzeitig ab: Primär reagiert Ethen mit sich selbst. Sekundäre Reaktionen erfolgen zwischen Ethen und bereits gebildeten Ethen-Oligomeren oder Oligomeren untereinander. Verglichen mit der Polymerisierung entstehen bei der Oligomerisierung Moleküle mit einer deutlich geringeren Anzahl an Kohlenstoffatomen. Die Grenze kann bei sechszehn Kohlenstoffatomen gesetzt werden. Wichtiges Merkmal einer Oligomerisierung ist auch, dass aus Olefinen wiederum neue Olefine gebildet werden und keine gesättigten Ketten.

Nach alldem ist unter einer Ethen-Oligomerisierung im Sinne dieser Erfindung die chemische Reaktion von Ethen zu verstehen unter der Bildung von Olefinen mit einer Kohlenstoff-Anzahl von vier bis sechszehn.

Die Ethen-Oligomerisierung wird industriell praktiziert zur Herstellung von C₄-, C₆- und C₈-Olefinen. Diese dienen wiederum als Edukt für komplexere Chemikalien, wie beispielsweise höhere Alkohole, Carbonsäuren und Esther.

Aus verfahrenstechnischer Sicht lassen sich Oligomerisierungs-Prozesse unterscheiden zwischen solchen, die in der Gasphase durchgeführt werden und solchen, die in der flüssigen Phase erfolgen. Des Weiteren unterscheidet man heterogen katalysierte Prozesse von homogen katalysierten Verfahren.

In Gasphasen-Prozessen wird die Oligomerisierung bei Bedingungen durchgeführt, in denen Ethen gasförmig ist. Die Oligomere können dann ebenfalls gasförmig sein oder auch flüssig.

Bei der Flüssigphasen-Oligomerisierung wird das Ethen in flüssiger Form in den Reaktor eingebracht. Da Ethen nur unter sehr großem Druck flüssig ist, wird die Flüssigphasen-Oligomerisierung von Ethen meist dadurch realisiert, dass gasförmiges Ethen in einem flüssigen Lösemittel gelöst wird und die Oligomerisierung in dem flüssigen Lösemittel erfolgt. Die Oligomere finden sich dann auch in dem Lösemittel. Vorteil der Flüssigphasen-Oligomerisierung gegenüber der Gasphasen-Oligomerisierung ist die bessere Ausnutzung des Reaktorvolumens und eine bessere Abfuhr der Reaktionswärme mit dem Lösemittel (Die Oligomerisierung ist stark exotherm!). Insgesamt erzielt die Flüssigphasen-Oligomerisierung eine bessere Prozess-Intensität als die Gasphasen-Oligomerisierung.

Nachteil der Flüssigphasen-Oligomerisierung ist die Notwendigkeit eines Lösemittels und die schwierige Produktabtrennung: Produktabtrennung ist die Gewinnung der Oligomere aus dem Reaktionsgemisch, das neben den gewünschten Oligomeren auch nicht umgesetztes Ethen, ggf. Lösemittel und Katalysator umfasst. Sofern der Prozess homogen katalysiert ist, liegt der Katalysator gelöst in derselben Phase vor wie das Edukt und das Oligomerisat. Sofern der Katalysator nicht in dem Produktgemisch verbleiben kann, muss er entsprechend abgetrennt werden. Dies bereitet zusätzlichen verfahrenstechnischen Aufwand.

Einfacher ist hingegen die Katalysatorabtrennung bei heterogen katalysierten Prozessen, bei denen der Katalysator in einer anderen Phase vorliegt als die Reaktanden, in der Regel als ein Festkörper. Sowohl bei der heterogen katalysierten Flüssigphasen-Oligomerisierung, als auch bei der heterogen katalysierten Gasphasen-Oligomerisierung verbleibt der feste Katalysator in dem Reaktor, während das fluide Produktgemisch aus dem Reaktor abgezogen wird.

Die Tatsache, dass der heterogene Katalysator im Reaktor verbleibt, ermöglicht es, ihn ständig wiederzuverwenden. Dies hat allerdings zur Folge, dass der Katalysator mit steigender Nutzungsdauer an Aktivität verliert und zunehmend unerwünschte Nebenprodukte bildet. Dies ist wohl darauf zurückzuführen, dass die aktiven Zentren des Katalysators mit Ablagerungen belegt werden, sodass kein Kontakt mehr zu dem Ethen besteht. Bei diesen Ablagerungen handelt es sich wahrscheinlich um längerkettige Nebenprodukte bis hin zu niedermolekularem Polyethylen und/oder um Katalysatorgifte bestehend aus N-, O- oder S- haltigen Molekülen. Auch können in der Oligomerisierung aktive Metalle des Katalysators oxidiert werden und damit ihre Wirkung verlieren.

Glücklicherweise ist die Desaktivierung heterogener Oligomerisierungs-Katalysatoren reversibel: So ist es möglich, den Katalysator von Zeit zu Zeit zu reaktivieren, wodurch er seine anfängliche Performance weitestgehend zurück erlangt.

Im Stand der Technik sind einige Verfahren zur Reaktivierung von Oligomerisierungs-Katalysatoren beschrieben:
So beschreibt DE102009027408A1 die Regeneration eines heterogenen Katalysators auf Basis von Nickeloxid, Siliciumdioxid und Aluminiumdioxid. Er wird bevorzugt in der Oligomerisierung von C₃- bis C₆-Oelfinen eingesetzt. Zur Entfernung organischer Ablagerungen werden diese mit einem heißen, sauerstoffhaltigen Gasstrom abgebrannt. Dies geschieht in einem Ofen. Nachteil dieses Verfahrens ist, dass der Katalysator zur Reaktivierung aus dem Reaktor ausgebaut und in den Ofen gebracht werden muss. Nach erfolgter Regeneration muss der Katalysator zurück in den Reaktor eingebaut werden. Dies ist mit vergleichsweise viel manueller Arbeit verbunden und
verursacht lange Stillstandzeiten der Oligomerisierungs-Anlage.

Einfacher ist da schon die in EP0136026B1 beschriebene Regeneration eines in der gemischten Oligomerisierung von C₂- und C₃-Olefinen eingesetzten, heterogenen Zeolith-Katalysators. Hier erfolgt die Regeneration in-situ, also an dem Ort, an dem der Katalysator normalerweise eingesetzt wird, nämlich im Reaktor. Zur Regeneration selbst wird der Olefin-Strom auf den Katalysator abgeschaltet und der Reaktor mit dem darin enthaltenden Katalysator mit einem heißen, oxidierenden Gas gespült. Die Regeneration in-situ hat den entscheidenden Vorteil, dass der Katalysator zur Regeneration nicht aus dem Reaktor ausgebaut und danach wieder eingebaut werden muss. Nachteil dieses Verfahrens ist, dass der Reaktor thermisch so stabil ausgelegt werden muss, dass er auch den heißen Regenerationsgasen standhält. Solange der Reaktor ohnehin für die Gasphasen-Oligomerisierung ausgelegt ist - wie dies in EP0136026B1 der Fall ist - sind die Mehrkosten vertretbar. Einen für die Flüssigphasen-Oligomerisierung optimierten Reaktor für die Regeneration mit heißen Gasen zu ertüchtigen ist hingegen mit deutlichen Mehrkosten verbunden. Darüber hinaus ist es fraglich, ob sich alle heterogenen Katalysatoren mit Heißgas regenerieren lassen, denn die Beispiele der EP0136026B1 befassen sich lediglich mit zeolithischen Katalysatoren. Insbesondere solche Katalysatoren, die als aktives Metall Nickel, Chrom, Eisen oder Titan enthalten, verhalten sich in der Oligomerisierung völlig anders als die nur aus Aluminium- und Siliciumoxiden bestehenden Zeolithe. Es ist daher zu erwarten, dass solche Katalysatoren einer anderen Regeneration bedürfen.

Die Regeneration eines Chrom-basierten Katalysators, der in der Flüssigphasen-Oligomerisierung von Ethen eingesetzt wird, ist in WO2014082689A1 beschrieben. Allerdings handelt es sich hierbei um ein homogenes Katalysatorsystem, welches ex-situ, also außerhalb des Reaktors regeneriert wird. WO2011136765A1 offenbart ein Verfahren zur Olefinoligomerisation, wobei die Katalysatorregenerierung zweistufig durch Erhitzen in einer Inertgasatmosphäre und in einer trockenen sauerstoffhaltigen Atmosphäre erfolgt. Es finden sich im Stand der Technik auch Beispiele der in-situ-Regeneration von Katalysatoren, die in der Flüssigphasen-Oligomerisierung von Ethen eingesetzt werden (WO2011112184A1, WO2010110801A1), allerdings sind dies auch alles homogene Systeme.

Die in-situ Regeneration von heterogenen Katalysatoren, die in der Flüssigphasen-Oligomerisierung von Ethen eingesetzt werden, ist nach derzeitigem Wissenstand bisher nicht beschrieben.

Deswegen ist es Aufgabe der Erfindung ein Verfahren zur Oligomerisierung von Ethen anzugeben, welches die Vorteile eines Flüssigphasen-Prozesses bietet und welches mit einem wiederverwertbaren, heterogenen Katalysator arbeitet, der sich mit geringem Aufwand regenerieren lässt.

Gelöst wird diese Aufgabe durch ein kombiniertes Verfahren zur Oligomerisierung von Ethen an einem heterogenen Katalysator und zur Regeneration dieses Katalysators, welches die folgenden Merkmale aufweist:
a) die Oligomerisierung erfolgt in einem Oligomerisierungs-Betrieb, in welchem Ethen, welches zumindest teilweise in einem flüssigen Lösemittel gelöst ist, mit dem heterogenen Katalysator kontaktiert wird;
b) die Regeneration erfolgt in einem Regenerations-Betrieb, in welchem der Katalysator in Abwesenheit von Ethen, Wasserstoff und Sauerstoff mit einem flüssigen Spülmedium gespült wird;
c) über die Zeit wird gewechselt zwischen dem Oligomerisierungs-Betrieb und dem Regenerations-Betrieb, sodass sich an einen zeitlich begrenzten Oligomerisierungs-Betrieb ein zeitlich begrenzter Regenerations-Betrieb anschließt, und an diesen wieder ein zeitlich begrenzter Oligomerisierungs-Betrieb;
d) der heterogene Katalysator befindet sich stets an demselben Ort, weswegen sowohl Oligomerisierungs-Betrieb als auch Regenerations-Betrieb an diesem Ort stattfinden;
e) der Ort, an dem sich der Katalysator befindet, wird mit Wärmenergie beaufschlagt, um ihm eine Vorgabe-Temperatur aufzuprägen, wobei die tatsächliche Temperatur am Katalysator zeitlich und örtlich begrenzt durchaus von der Vorgabe-Temperatur abweichen kann;
f) die Vorgabe-Temperatur im Regenerations-Betrieb ist höher als die Vorgabe-Temperatur im Oligomerisierungs-Betrieb.

Da sich bei diesem Verfahren der heterogene Katalysator stets an demselben Ort befindet, genauer gesagt, in dem Reaktor, handelt es sich bei der vorliegenden Regeneration um eine in-situ Regeneration des Katalysators im Reaktor. Dies hat den Vorteil, dass der Katalysator nicht aus dem Reaktor ausgebaut werden muss.

Ein weiterer wesentlicher Gesichtspunkt dieser Erfindung ist, dass sowohl die Oligomerisierung, als auch die Regeneration in der flüssigen Phase erfolgen: Die Regeneration erfolgt nämlich mit Hilfe eines flüssigen Spülmediums, mit welchem die Ablagerungen von dem Katalysator abgelöst und aus dem Reaktor ausgetragen werden. Um die Ablösung zu ermöglichen, wird das Spülmedium bei einer gegenüber der Oligomerisierung erhöhten Temperatur durchgeführt. Freilich bedeutet dies, dass der Reaktor eine höhere thermische Beständigkeit haben muss; allerdings ist der damit verbundene apparative Mehraufwand - verglichen mit einem Flüssigphasen-Reaktor, der mit Heißgas gespült werden muss - deutlich geringer.

Schließlich ist ein wichtiger Aspekt der Erfindung, dass die Regeneration in Abwesenheit von Ethen erfolgt. Es wird also nicht im laufenden Oligomerisierungs-Betrieb regeneriert sondern zeitlich davon versetzt im Regenerations-Betrieb. Erfindungsgemäß wird also zwischen einem Oligomerisierungs-Betrieb und einem Regenerations-Betrieb gewechselt. Diese beiden Betriebsarten sind als sich abwechselnde Zeitphasen zu verstehen.

Darüber hinaus erfolgt die Regeneration auch in Abwesenheit von Wasserstoff oder Sauerstoff. Der Katalysator bzw. seine Ablagerungen werden also nicht oxidiert. Wie bereits erwähnt lässt sich Ethen nur bei großem Druck verflüssigen. Um es bei geringem Druck in der flüssigen Phase zu verarbeiten, wird das an sich gasförmige Ethen in einem Lösemittel gelöst, welches wiederum bei geringem Druck flüssig ist. Grundsätzlich kann ein inertes Lösungsmittel verwendet werden oder ein reaktives. Unter einem inerten Lösemittel ist zu verstehen, dass es sich in der Ethen-Oligomerisierung inert verhält, also nicht chemisch reagiert. Ein reaktives Lösemittel reagiert in der Oligomerisierung mit. Als inerte Lösemittel kommen insbesondere Alkane (Paraffine) mit drei bis zehn Kohlenstoffatomen pro Molekül in Betracht. Diese Alkane haben einen höheren Siedepunkt als Ethen und sind deswegen bei den angestrebten Oligomerisierungsbedingungen flüssig. Aufgrund des abweichenden Siedepunkts lassen sie sich auch gut von Ethen und den Oligomeren abtrennen. Hinsichtlich der genauen Siedepunktlage des gewählten Lösemittels im Verhältnis zu den Oligomeren wird auf die zum Zeitpunkt der Anmeldung noch unveröffentlichten europäischen

Patentanmeldungen 15151624.2 und 15151621.8 vom 19.01.2015 verwiesen.

Es sei in diesem Zusammenhang klargestellt, dass sich auch die zyklischen Alkane mit drei bis zehn Kohlenstoffatomen als Lösemittel eignen und dass selbstverständlich auch Mischungen mehrerer Alkane/Cycloalkane als Lösemittel eingesetzt werden können.

Als reaktive Lösemittel kommen Olefine (Alkene) in Betracht mit drei bis zwölf Kohlenstoffatomen oder Mischungen daraus. Diese Olefine reagieren in der Oligomerisierung mit, sodass ein Oligomerisat mit einem breiteren Produktspektrum gebildet wird. Man nennt dies Co-Oligomerisierung. Das Lösemittel kann auch ein Gemisch aus reaktiven und inerten Substanzen sein.

Für die Auswahl des Spülmediums gelten dieselben Kriterien. Überraschenderweise hat sich gezeigt, dass mit denselben Substanzen, die als Lösemittel taugen, auch der Katalysator regeneriert werden kann. Eine besondere Ausführungsform der Erfindung sieht demnach vor, als Lösemittel und Spülmedium die identische Substanz bzw. das identische Gemisch zu verwenden. Der Unterschied besteht dann nur darin, dass im Oligomerisierungs-Betrieb dem Lösemittel das Ethen zudosiert wird, währenddessen im Regenerations-Betrieb das Lösemittel ohne Ethen bei höherer Temperatur als Spülmedium verwendet wird. Die Verwendung des identischen Lösemittels/Spülmediums hat den Vorteil, dass beim Wechsel von Oligomerisierungs-Betrieb in den Regenerations-Betrieb lediglich das Ethen abgeschaltet und die Temperatur erhöht werden muss. Dies macht den Betriebszustandswechsel sehr einfach und automatisierbar.

Die folgenden inerten Substanzen bzw. deren Gemische haben sich sowohl in der Funktion als Lösemittel, als auch in der Funktion als Spülmedium als besonders geeignet erwiesen: Propan, Isobutan, Pentan, Cyclopentan, Hexan, Cyclohexan, Heptan, Cycloheptan.

Ein wesentlicher Aspekt der Erfindung ist, dass die Vorgabe-Temperatur im Regenerations-Betrieb höher ist als die Vorgabe-Temperatur im Oligomerisierungs-Betrieb. Im Oligomerisierungs-Betrieb sollte die Vorgabe-Temperatur zwischen 20 °C und 130 °C liegen, im Regenerations-Betrieb zwischen 80 °C und 150 °C. Wenngleich sich diese Intervalle überlappen, gilt weiterhin die Maßgabe, dass die Vorgabe-Temperatur im Regenerationsbetrieb höher gewählt ist als die Vorgabe-Temperatur im Oligomerisierungs-Betrieb. Es können also nicht beliebige Temperatur-Paare aus beiden Intervallen zusammengestellt werden.

Noch eine Anmerkung zu den Vorgabe-Temperaturen: Wie bereits erwähnt, ist die Oligomerisierung von Ethen stark exotherm. Daher werden bei der Oligomerisierung mitunter Temperaturen erreicht, die oberhalb der Vorgabe-Temperatur liegen. Deswegen muss der Ort, an dem die Oligomerisierung stattfindet (also der Reaktor) im Oligomerisierungs-Betrieb auf die Vorgabe-Temperatur gekühlt werden. Nichtdestotrotz kann es vorkommen, dass es innerhalb des Reaktors, und dort auch lokal begrenzt, zu Temperaturspitzen oberhalb 130 °C kommt. Die tatsächliche Reaktionstemperatur kann also von der Vorgabe-Temperatur durchaus abweichen. Sofern ein inertes Spülmedium verwendet wird, findet im Regenerations-Betrieb keine spürbare Reaktion statt, sodass zur Einhaltung der Vorgabe-Temperatur im Regenerations-Betrieb geheizt werden muss. Wird jedoch ein reaktives Spülmedium verwendet, etwa Buten, oligomerisiert dieses beim Spülen, sodass zur Erhöhung der Vorgabe-Temperatur im Regenerations-Betrieb die Reaktionswärme aus dem Spülmedium verwendet werden kann.

Die Betriebsbedingungen werden vorzugsweise wie folgt gewählt:

**Oligomerisierungs-Betrieb:**

| | |
|---|---|
| Druck: | 1*10⁵ Pa bis 50*10⁵ Pa |
| WHSV: | 2 h⁻¹ bis 50 h⁻¹ |
| Ethen-Anteil in Gesamtlösung: | 1 Gew.-% bis 50 Gew.-% |

**Regenerations-Betrieb:**

| | |
|---|---|
| Druck: | 1*10⁵ Pa bis 50*10⁵ Pa |
| WHSV: | 2 h⁻¹ bis 50 h⁻¹ |
| Ethen-Anteil in Gesamtlösung: | 0 Gew.-% bis 1 Gew.-% |

Die Drücke sind dabei in Hinblick auf die im Reaktor herrschenden Temperaturen so zu wählen, dass das Lösemittel bzw. das Spülmedium im flüssigen Zustand vorliegt.

Im Oligomerisierungs-Betrieb wird der Ethen-Anteil in Abhängigkeit von Druck und Temperatur vorzugsweise so gewählt, dass das Ethen vollständig in der flüssigen Phase gelöst ist. Es ist aber auch möglich, mehr Ethen zu dosieren, sodass ein Teil des Ethens in der flüssigen Phase gelöst ist und ein Teil des Ethens nicht in dem Lösemittel gelöst ist, sondern daneben eine Gasphase bildet. Vorzugsweise werden Druck und spezifische Katalysatorbelastung (weight hourly space velocity, WHSV) identisch gewählt, sodass es beim Umschalten zwischen den Betriebszuständen kaum Lastwechsel gibt. Dies bietet sich insbesondere dann an, wenn Spülmedium und Lösemittel identisch sind. Oligomerisierungs-Betrieb und Regenerations-Betrieb unterscheiden sich dann praktisch nur durch den Ethen-Gehalt in der Flüssigkeit und durch die Vorgabe-Temperatur.

Im Übrigen lässt sich der Katalysator rascher reaktivieren als er desaktiviert. Deswegen lässt sich die Dauer einer Regenerations-Phase deutlich kürzer bemessen als die Dauer einer Produktions-Phase. In der Praxis sollte die Dauer des Regenerations-Betriebs weniger als 20 %, bevorzugt weniger als 10 % und ganz besonders bevorzugt weniger als 5 % der Dauer des vorhergehenden Oligomerisierungs-Betriebs betragen. Die Regenerationszeiten können durch Wahl einer besonders hohen Vorgabe-Temperatur im Regenerations-Betrieb verkürzt werden. Allerdings ist die Temperatur nicht so hoch zu wählen, dass das Spülmedium siedet. Es muss sichergestellt sein, dass flüssig gespült wird.

Der Ort, an dem sich der Katalysator befindet und an dem der Regenerations-Betriebs und der Oligomerisierungs-Betriebs erfolgen, ist im weitesten Sinne ein Reaktor, also ein Apparat, der einen kontrollierten Stoff- und Wärmeaustausch mit seiner Umgebung zulässt. Technisch realisiert wird dies meist in der Bauweise eines Rohrbündel-Reaktors, der mehrere parallel geschaltete Einzelreaktoren umfasst. Es können auch mehrere Reaktoren hintereinander geschaltet sein. Für die Erfindung kommt es nicht darauf an, ob ein einzelner oder mehrere Apparate als Reaktor verwendet werden. Wesentlich ist lediglich, dass sich der Katalysator stets an demselben abgeschlossen Ort befindet, der eben einen kontrollierten Stoffaustausch und Wärmeaustausch mit der Umgebung ermöglicht. Innerhalb dieses Ortes kann der Katalysator aber durchaus bewegt werden, etwa zwischen einer Reaktionszone und einer Regenerationszone hin und her, solange für beide Zonen derselbe Stoff- und Wärmeaustausch mit der Umgebung geschaffen wird. Es ist auch möglich, zwei oder drei parallel geschaltete Reaktoren an einem Ort aufzustellen, von denen sich einer im Regenerations-Betrieb und ein anderer zeitgleich im Oligomerisierungs-Betrieb befindet. Ein dritter kann sich im Stand-by-Betrieb befinden. Zum Wechsel der Betriebszustände wird die Funktion der einzelnen Reaktoren revolvierend getauscht.

Der in diesem Zusammenhang erwähnte Stoffaustausch bedeutet im Regenerations-Betrieb, dass der Katalysator vor Ort mit frischem Spülmedium beaufschlagt wird, und dass dieses nach dem Kontakt mit dem Katalysator wieder von dem Ort abgezogen wird. Der Katalysator kommt also ausschließlich mit dem Spülmedium in Kontakt. Insbesondere kommt der Katalysator weder mit Wasserstoff noch mit Sauerstoff in Kontakt, da beides nicht im Spülmedium enthalten ist. Der Katalysator wird also in der Regeneration nicht oxidiert. Der Katalysator kann kontinuierlich gespült werden oder im Batch-Betrieb, wobei die kontinuierliche Regeneration bevorzugt ist.

Analog bedeutet Stoffaustausch im Oligomerisierungs-Betrieb, dass der feste Katalysator mit dem flüssigen Ethen bzw. mit dem im flüssigen Lösemittel gelösten Ethen beaufschlagt wird und das Oligomerisat von dem Ort, an dem sich der Katalysator befindet, abgezogen wird, während der Katalysator vor Ort verbleibt. Der Katalysator kommt also nur mit dem Ethen, bzw. mit dem Lösemittel bzw. mit dem Oligomerisat in Kontakt. Der Oligomerisierungs-Betrieb ist ebenso kontinuierlich und Batch-weise möglich, wobei eine kontinuierliche Betriebsweise bevorzugt ist.

Da erfindungsgemäß die Vorgabe-Temperatur im Regenerations-Betrieb erhöht wird, ist die Beherrschung des Wärmeaustauschs zwischen dem Ort, an dem sich der Katalysator befindet, und seiner Umgebung für das Verfahren erfolgsmaßgeblich. Da im Oligomerisierungs-Betrieb Wärme von dem Ort abgeführt und im Regenerations-Betrieb Wärme zugeführt werden muss, wird der Ort einfachstenfalls dadurch temperiert, dass von einem Temperier-Medium durchflossen ist, und dass die Vorgabe-Temperatur durch die Vorlauf-Temperatur des Temperier-Mediums vorgegeben wird. Je nachdem, wie die Vorlauf-Temperatur des Temperier-Mediums gewählt wird, wird der Ort mit positiver Wärmeenergie oder negativer Wärmeenergie beaufschlagt, also beheizt oder gekühlt. Abhängig von der zu übertragenden Wärmeenergie und der Wärmekapazität des Temperier-Mediums ist auch der Massenstrom des Temperier-Mediums entsprechend anzupassen. Als Temperier-Medium wird einfachstenfalls flüssiges Wasser oder organisches Öl oder Silicon-Öl verwendet, da diese Medien eine hohe Wärmekapazität aufweisen. Im Heizbetrieb zum Regenerieren kann auch Wasserdampf verwendet werden. Zwischen Temperier-Medium und den Reaktanden kommt es nicht zu einem Stoffaustausch, lediglich ein Wärmeaustausch ist insoweit beabsichtigt. Demzufolge ist der Reaktor mit einem Wärmetauscher zu versehen, der die Wärmeenergie zwischen Reaktanden und Temperier-Medium austauscht.

Alternativ oder ergänzend kann die Wärmeenergie über das Lösemittel bzw. das Spülmedium in den Reaktor eingetragen bzw. ausgetragen werden. Zu diesem Zwecke wird das Lösemittel bzw. das Spülmedium vor Eintritt in den Reaktor gekühlt oder geheizt. Sofern ein reaktives Löse- oder Spülmedium verwendet wird, kann die erforderliche Wärmeenergie auch durch chemische Reaktion des Lösemittels bzw. des Spülmediums direkt am Katalysator freigesetzt bzw. aufgenommen werden.

Die Beheizung kann auch elektrisch erfolgen, dies ist aber energetisch nicht sinnvoll.

In der Gesamtschau lässt sich die Temperatur am effektivsten und effizientesten über ein gesondertes Temperier-Medium beherrschen.

Eine bevorzugte Variante der Verfahrensführung sieht vor, dass im Regenerations-Betrieb das Spülmedium im Kreislauf geführt wird, und dass das im Kreislauf geführte Spülmedium außerhalb des Ortes, an dem sich der Katalysator befindet, mit Hilfe eines Trennapparats von zumindest einem Teil von im Spülmedium gelösten Komponenten gereinigt wird. Dies hat den Vorteil, dass das Spülmedium wiederverwertet werden kann. Die Kreislauf-Fahrweise macht vor allem dann Sinn, wenn ein inertes Spülmedium verwendet wird, da reaktive Spülmedien ja am Katalysator verbraucht werden. In der Kreislauffahrweise sind vor allem die Alkane bzw. Cycloalkane mit drei bis zehn Kohlenstoffatomen oder Mischungen daraus als Spülmedium geeignet.

Bei dem Trennapparat handelt es sich vorzugsweise um einen Filter oder um eine Membran oder um eine Kühlfalle oder um eine Kombination mehrerer dieser Trennapparate. Solche Trennapparate sind allgemein erhältlich und geeignet, die von dem Katalysator abgelösten Verunreinigungen wieder aus dem Spülmedium zu entfernen, sodass das gereinigte Spülmedium wieder rezykliert werden kann. Die Kühlfalle ist besonders bevorzugt, da die typischen Verunreinigungen besonders gut ausgefroren werden können. Auch verstopft sie nicht so leicht wie ein Filter oder eine Membran. Die Kühlfalle arbeitet vorzugsweise bei einer Temperatur unter 75 °C. Oberhalb von 80 °C lassen sich nämlich die Ablagerungen nicht mehr ausfrieren. Nach unten kann die Arbeitstemperatur auf Umgebungstemperatur begrenzt sein, also auf 20 °C. Die Kühlfalle weiter herab zu kühlen macht keinen Sinn, da die meisten Ablagerungen bereits bei etwa 70 °C aus dem Spülmedium ausfallen.

Sofern ein Trennapparat vorgesehen ist, macht es Sinn, diesen unmittelbar stromabwärts hinter dem Reaktor anzuordnen. Dadurch wird verhindert, dass gelöste Ablagerungen in nachgelagerte Produktionseinrichtungen eingetragen werden und sich dort absetzen. Darüber hinaus sollte in Betracht gezogen werden, auch im Oligomerisierungs-Betrieb den Reaktoraustrag, also das Oligomerisat, durch den Trennapparat zu fahren, da nicht ausgeschlossen werden kann, dass bereits im Oligomerisat Stoffe gelöst sind, die sich auf dem Katalysator oder in anderen Anlagenkomponenten ablagern könnten, insbesondere in Kreislauf-Fahrweise. Die Reinigung des Reaktor-Austrags im laufenden Oligomerisierungs-Betrieb könnte die Zeit bis zur nächsten Regeneration verlängern.

Wie bereits erwähnt, bietet sich die Regeneration in der Kreislauf-Fahrweise vor allem dann an, wenn ein inertes Spülmedium verwendet wird. Wird ein zumindest teilweise reaktives Spülmedium eingesetzt, sollte das Spülmedium nach der Regeneration des Katalysators in eine andere Reaktion gefahren werden. Eine entsprechende Variante des Verfahrens, bei der es sich bei dem Spülmedium um ein Alken mit drei bis zwölf Kohlenstoffatomen handelt oder um eine Mischung mehrerer solcher Alkene oder um eine Mischung eines oder mehrerer solcher Alkene mit mindestens einem Alkan mit drei bis sieben Kohlenstoffatomen, ist dadurch gekennzeichnet, dass im Regenerations-Betrieb das Spülmedium von dem Ort, an dem sich der Katalysator befindet, abgezogen und - erforderlichenfalls nach einer Reinigung - auf einen zweiten heterogenen Katalysator geführt wird, der sich entfernt von dem zu regenerierenden Katalysator befindet, und dass mindestens ein im Spülmedium enthaltendes Alken an dem zweiten Katalysator einer chemischen Reaktion unterworfen wird. Das benutzte Spülmedium wird nicht zu dem in der C2-Oligomerisierung eingesetzten Katalysator rezykliert sondern an dem zweiten Katalysator, in einer zweiten Reaktion verwertet. Die zweite Reaktion an dem zweiten Katalysator muss also in der Lage sein, das Spülmedium umzusetzen - möglichst ohne Schaden an den darin enthaltenden Verunreinigungen zu nehmen. Sofern die in dem Spülmedium gelösten Ablagerungen vom regenerierten Katalysator den zweiten Katalysator vergiften könnten, ist das gebrauchte Spülmedium vor Eintritt in die zweite Reaktion entsprechend zu reinigen. Dies kann in derselben Weise erfolgen wie das im Kreislauf-Verfahren geführte Spülmedium gereinigt wird, etwa mit einer Kühlfalle oder einem anderen Trennapparat. Sofern es sich um ein olefinisches Spülmedium handelt, kann als zweite Reaktion insbesondere eine zweite Oligomerisierung, eine Isomerisierung oder eine Veretherung oder eine oxidative Dehydrierung vorgesehen sein. Wird etwa als Spülmedium ein Gemisch enthaltend Butene genutzt, können die Butene nach erfolgter Regeneration des in der C₂-Oligomerisierung eingesetzten Katalysators zu Methyl-tert.-butylether (MTBE) verethert werden oder oxidativ zu 1,3-Butadien dehydriert werden, oder an einem für die Oligomerisierung von Butenen geeigneten Katalysator zu C₈- oder C₁₂- Olefinen oligomerisiert werden.

Die verglichen mit den Alkenen reaktionsträgen Alkane verhalten sich in der Oligomerisierung praktisch inert. Dennoch gibt es chemische Reaktionen, in denen sich die Alkane nicht inert verhalten und die deswegen geeignet sind, ein paraffinisches Spülmedium zu verwerten. Dies sind insbesondere pyrolytische Dehydrierungen und Spaltung von Alkanen zu Alkenen (das so genannte Cracken). In einem gesonderten Schritt bestünde dann noch die Möglichkeit, der Addition von Wasser an das Alken zum Alkohol und anschließende Oxidation zur Carbonsäure. Schließlich kann das paraffinische Spülmedium noch verbrannt und damit thermisch verwertet werden. Folglich ist eine Variante des Verfahrens, bei welcher es sich bei dem Spülmedium um ein Alkan mit drei bis sieben Kohlenstoffatomen handelt oder um eine Mischung mehrerer solcher Alkane, dadurch gekennzeichnet, dass im Regenerations-Betrieb das Spülmedium von dem Ort, an dem sich der Katalysator befindet, abgezogen und auf einen zweiten heterogenen Katalysator geführt wird, der sich entfernt von dem zu regenerierenden Katalysator befindet, und dass mindestens ein im Spülmedium enthaltendes Alkan an dem zweiten Katalysator einer chemischen Reaktion unterworfen wird.

Der im erfindungsgemäßen Verfahren eingesetzte Katalysator liegt nicht in der flüssigen Reaktionsphase vor und ist deswegen als heterogen zu bezeichnen. Genauer gesagt, handelt es sich bei dem Katalysator um einen Feststoff, der von dem flüssigen Reaktionsgemisch umspült ist. Zur Oligomerisierung von Ethen geeignet und regenerierbar sind insbesondere solche Katalysatoren, die aus mindestens zwei Komponenten bestehen, wobei die erste Komponente mindestens ein aus Ni, Cr, Fe, Ti ausgewähltes Element umfasst, welches in metallischer und/oder oxidischer und/oder hydridischer Form vorliegt, und wobei die zweite Komponente mindestens ein aus Al₂O₃, SiO₂, TiO₂, ZrO₂ ausgewähltes Metalloxid umfasst. Ein Beispiel eines solchen Katalysators ist aus US2581228 bekannt.

Die Oligomerisierung wird vorzugsweise so gefahren, dass weniger als 5 Gew.-% des umgesetzten Ethens in Oligomere bzw. Polymere des Ethens mit sechszehn oder mehr als sechszehn Kohlenstoffatomen umgesetzt werden. Dies bedeutet, dass überwiegend Dimere (C₄-Olefine), Trimere (C₆-Olefine) und Tetramere (C₈-Olefine) gebildet werden, daneben noch ein paar wenige C₁₀-er und C₁₂-er. Vorzugsweise werden C₄-, C₆- und C₈-Olefine mit einer Gesamt-Selektivität größer 90 % gebildet. Die höheren Oligomere des Ethens bzw. Kettenmoleküle aus Polyethylen sind unerwünschte Nebenprodukte. Insoweit unterscheidet sich die C₂-Oligomerisierung von der Polyethylen-Herstellung, die weitaus größere Kettenlängen anstrebt und auch keine Olefine bildet. Eine bevorzugte Ausführungsform des Verfahrens sieht vor, dass der Umsatz des Ethens im Oligomerisierungs-Betrieb kontinuierlich bestimmt wird, und dass dann von dem Oligomerisierungs-Betrieb in Regenerations-Betrieb gewechselt wird, wenn der Umsatz des Ethens auf einen Wert zwischen 95 % und 100 % gefallen ist.

Die dahinter stehende Idee besteht darin, dass mit der Regeneration nicht so lange gewartet wird, bis der Katalysator bereits sehr stark desaktiviert ist, sondern dass schon relativ früh, wenn erste Umsatzeinbußen feststellbar sind, in den Regenerations- Betrieb gewechselt wird. Früh bedeutet in diesem Zusammenhang, wenn der Umsatz an Ethen von den optimalen 100 % auf einen Wert zwischen 95 % und 100 % gefallen ist. Optimal wird bei etwa 97 % Umsatz in den Regenerations-Betrieb gewechselt. Das frühzeitige Wechseln führt insgesamt zu einem gepulsten Regenerations-Betrieb, also zu relativ häufigen, aber kurzen Regenerationsphasen. Langzeitversuche deuten darauf hin, dass bei gepulsten Regenerationsphasen der Katalysator deutlich längere Standzeiten erreicht und der Umsatz über längere Zeiträume bei annährend Vollumsatz (100 %) gefahren werden kann.

Die technische Umsetzung der Erfindung soll nun anhand von Verfahrensfließbildern und anhand von Versuchsergebnissen erläutert werden. Hierfür zeigen:
- Figur 1:: Verfahrensfließbild einer ersten Ausführungsform I im Oligomerisierungs-Betrieb;
- Figur 2:: Verfahrensfließbild der ersten Ausführungsform I im Regenerations-Betrieb;
- Figur 3:: Verfahrensfließbild einer zweiten Ausführungsform II im Oligomerisierungs-Betrieb;
- Figur 4:: Verfahrensfließbild der zweiten Ausführungsform II im Regenerations-Betrieb;
- Figur 5:: Reaktions- und Regenerationsphasen in Isobutan/Raffinat III (zu Beispiel 1);
- Figur 6:: Reaktions- und Regenerationsphasen in Isobutan pur (zu Beispiel 2);
- Figur 7:: Reaktions- und Regenerationsphasen im gepulsten Betrieb (zu Beispiel 3).

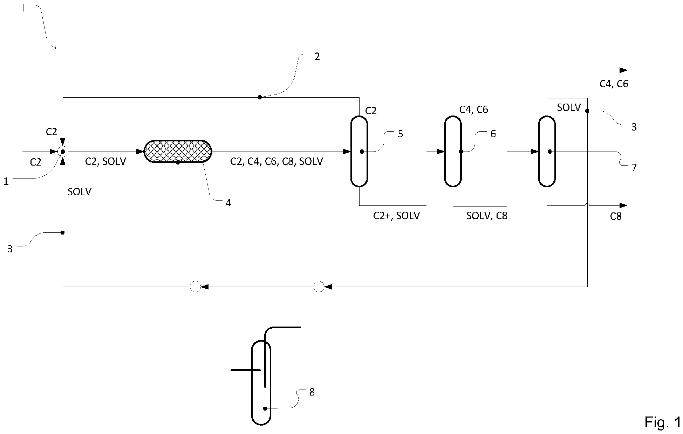

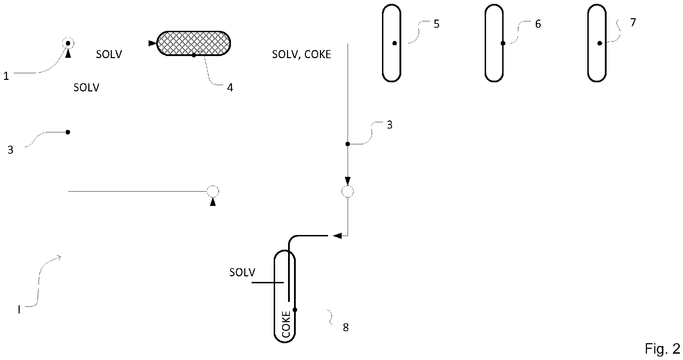

Ein Verfahrensfließbild zur Durchführung einer ersten Ausführungsform I der Erfindung ist in den Figuren 1 und 2 dargestellt, und zwar einmal im Oligomerisierungs-Betrieb und einmal im Regenerations-Betrieb. Diese Variante ist gekennzeichnet durch die Kreislauf-Fahrweise des Lösemittels bzw. des Spülmediums.

Im Oligomerisierungs-Betrieb (Figur 1) wird gasförmiges Ethen C2 in einem Mischer 1 mit einem Lösemittel SOLV zu einem Einsatzgemisch C2, SOLV vermischt. Bei dem Lösemittel handelt es sich beispielsweise um n-Heptan, einem C₇-Alkan. Das Ethen wird vollständig in dem Lösemittel gelöst, sodass das Einsatzgemisch C2, SOLV vollständig flüssig ist und sich darin keine Blasen aus Ethen-Gas bilden. Mischer 1 vermischt frisches Ethen aus einer nicht dargestellten Ethen-Quelle und rezykliertes Ethen aus einer Ethen-Rückführleitung 2. Das Lösemittel SOLV stammt aus einem Lösemittel-Kreislauf 3.

Das Einsatzgemisch C2, SOLV wird in einen Reaktor 4 gefahren, der mit einem festen heterogenen Katalysator gefüllt ist. In dem Reaktor, an dem Katalysator findet die Oligomerisierung des Ethens statt, und zwar innerhalb des Lösemittels. Dabei entsteht ein flüssiges Oligomerisat C2, C4, C6, C8, SOLV, welches hauptsächlich die Dimere des Ethens C₄, seine Trimere C₆ und seine Tetramere C₈ enthält. Des Weiteren enthält das Oligomerisat C2, C4, C6, C8, SOLV nicht umgesetztes Ethen C₂ und nicht umgesetztes Lösemittel SOLV. Das Ethen C2 ist nicht umgesetzt, da es im Überschuss im Einsatzgemisch enthalten war und die Verweilzeit im Reaktor 4 zu kurz war. Das Lösemittel wird nicht umgesetzt, da es inert ist. Schließlich enthält das Oligomerisat C2, C4, C6, C8, SOLV minimale Mengen höherer Oligomere und Polymere mit mehr als acht Kohlenstoffatomen. Dieser Anteil ist aber deutlich kleiner als 5 %.

Das Oligomerisat C2, C4, C6, C8, SOLV wird dann in eine erste (Destillations-)Kolonne 5 gefahren und darin das enthaltende überschüssige Ethen C2 über Kopf abgetrennt und über die Ethen-Rückführleitung 2 in den Mischer 1 zurückgeführt. Das überschüssige Ethen geht also nicht verloren. Im Sumpf der ersten Kolonne 5 bleiben die Olefine mit mehr als drei Kohlenstoffatomen C2+ sowie das Lösemittel SOLV.

In einer zweiten Kolonne 6 wird der Sumpf der ersten Kolonne destillativ getrennt in ein Kopfprodukt enthaltend die C4- und C6-Olefine und in ein Sumpfprodukt enthaltend das Lösemittel und die C8er.

Die Dimere C4 und Trimere C6 stellen die eigentlichen Wertprodukte der C2-Oligomeriserung dar und werden weiter aufgearbeitet (nicht dargestellt).

In einer dritten Kolonne 7 wird nun das Lösemittel SOLV über Kopf aus dem Sumpfprodukt der zweiten Kolonne destillativ abgetrennt und in den Lösemittelkreislauf 3 gegeben. Dieser führt an einer Kühlfalle 8 vorbei zurück zum Mischer 1.

Im Sumpf der dritten Kolonne bleiben die Tetramere (C8) sowie geringe Mengen höherer Oligomere. Je nach Bedarf kann das Sumpfprodukt der dritten Kolonne 7 noch weiter aufgearbeitet werden. Die darin enthaltenden C8-Olefine kommen neben den Dimeren C4 und den Trimeren C6 noch als drittes Zielprodukt in Betracht.

Mit der Zeit desaktiviert der im Reaktor 4 enthaltene Katalysator. Um ihn zu regenerieren, wird die Ethen-Zufuhr abgeschaltet und die Vorgabe-Temperatur des Reaktors 4 erhöht. Das Lösemittel SOLV wird weiter über den Lösemittelkreislauf 3 zirkuliert, jedoch nun über die Kühlfalle 8 und unter Umgehung der Kolonnen 5, 6, 7. Auf diese Weise nimmt die Anlage den in Figur 2 dargestellten Regenerations-Betrieb ein.

Im Regenerations-Betrieb dient das als Lösemittel SOLV genutzte n-Heptan nunmehr als Spülmedium. Es löst Ablagerungen COKE von dem Katalysator ab und trägt diese aus dem Reaktor heraus. In der Kühlfalle 8 werden die Ablagerungen COKE aus dem Spülmedium SOLV ausgefroren und bleiben in der Kühlfalle 8. Das gereinigte Spülmedium SOLV wird zum Reaktor 4 rezykliert. Nach erfolgter Regeneration wird wieder in den in Figur 1 dargestellten Oligomerisierungs-Betrieb umgeschaltet. Die Ablagerungen COKE werden aus der Kühlfalle entfernt.

Eine nicht gezeichnete Variante der ersten Ausführungsform I könnte darin bestehen, dass ein Lösemittel SOLV mit einem anderen Siedepunkt als n-Heptan verwendet wird. Dies hätte zur Folge, dass die Kolonnensequenz entsprechend geändert werden muss. Würde beispielsweise Propan als Lösemittel verwendet, müsste dieses über Kopf abgetrennt werden.

Eine weitere Variante könnte darin bestehen, dass die Kühlfalle 8 im Oligomerisierungs-Betrieb nicht umgangen wird (vgl. Figur 1) sondern das rückgeführte Lösemittel auch im Oligomerisierungs-Betrieb durch die Kühlfalle strömt (Lösemittelkreislauf 3 durch die Kühlfalle 8). Dies hätte den Vorteil, dass bereits in der laufenden Produktion etwaige Ablagerungen aus dem Lösemittel entfernt werden. Nachteil ist der Wärmeverlust in der Kühlfalle. Die Verwendung eines nicht thermisch arbeitenden Trennapparats - beispielsweise einer Membran - könnte insoweit Abhilfe schaffen. Allerdings ist zu erwarten, dass im laufenden Oligomerisierungs-Betrieb mit dem Lösemittel weitaus weniger Ablagerungen von dem Katalysator entfernt werden als im Regenerations-Betrieb, da die Temperatur niedriger ist. Anders als in den Figuren 1 und 2 dargestellt, kann die Kühlfalle 8 auch unmittelbar stromabwärts hinter dem Reaktor 4 angeordnet sein, um bereits im laufenden Oligomerisierungs-Betrieb Ablagerungen von den Kolonnen 5, 6, 7 fern zu halten. Allerdings geht dies mit einem Temperaturverlust einher, sodass die Wirtschaftlichkeit dieser alternativen Anordnung der Kühlfalle gut geprüft werden sollte.

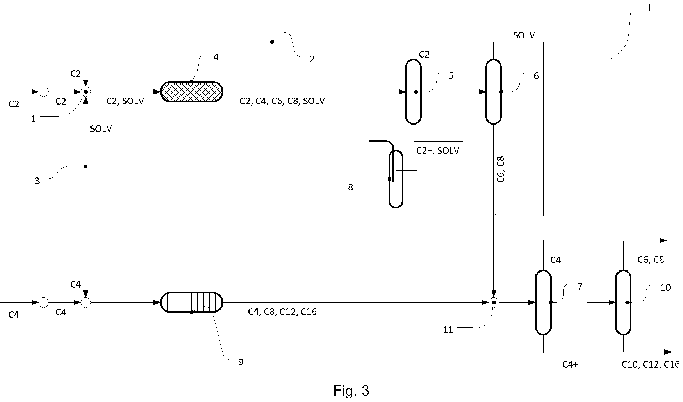

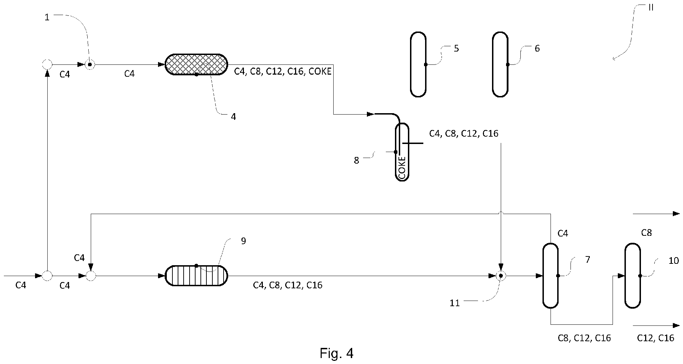

Eine zweite Ausführungsform II der Erfindung ist in den Figuren 3 und 4 dargestellt, wieder im Oligomerisierungs-Betrieb (Figur 3) und im Regenerations-Betrieb (Figur 4).

Die zweite Ausführungsform II ist ein Stück weit identisch mit der ersten Ausführungsform I, umfasst sie doch auch einen Mischer 1 zur Bereitstellung des Einsatzgemisches C2, SOLV, einen Reaktor 4 zur C₂-Oligomerisierung, eine erste Kolonne 5 zum Abtrennen des überschüssigen Ethens C2, welches über einen Ethen-Kreislauf 2 rezykliert wird und eine zweite Kolonne 6. Allerdings startet vom Kopf der zweiten Kolonne 6 bereits der Lösemittel-Kreislauf 3 zurück zum Mischer 1, da als Lösemittel SOLV in dieser Ausführungsform II ein Gemisch aus Isobutan und n-Buten verwendet wird. Die C4-Substanzen gehen schon an der zweiten Kolonne 6 über Kopf. Die höheren Oligomere C6, C8 vom Sumpf der zweiten Kolonne werden in einem zweiten Produktionsstrang aufgearbeitet, dazu später mehr.

Da es sich bei dem Lösemittel in der zweiten Ausführungsform II um ein Gemisch aus inertem Isobutan und reaktivem n-Buten handelt, reagiert im Reaktor ständig ein Teil des Lösemittels weg zu höheren Olefinen (C8, C12, C16), die letztendlich in den zweiten Produktionsstrang gelangen. Zugleich bildet aber die Dimerisierung von frischem Ethen n-Buten im Reaktor 4 nach, sodass der Anteil an n-Buten in dem Lösemittel grundsätzlich konstant gehalten werden kann aber nicht muss. So ist es auch möglich, den n-Buten-Anteil im Lösemittel zum Ende einer Oligomerisierungs-Phase gegen Null laufen zu lassen.

Der Grund, warum hier n-Buten zumindest teilweise als Lösemittel verwendet wird, ist, dass bei dieser Ausführungsform II zu der C2-Oligomerisierung parallel eine C4-Oligomerisierung in einem zweiten Reaktor 9 betrieben wird. Dieser setzt n-Buten C4 in der Flüssigphase an einem festen heterogenen Katalysator um, der sich von dem in der C2-Oligomerisierung eingesetzten Katalysator unterscheiden kann aber nicht muss.

In der C4-Oligomerisierung entsteht ein zweites Oligomerisat C4, C8, C12, C16, welches in einer dritten 7 und vierten Kolonne 10 destillativ aufgearbeitet wird. Die beiden Kolonnen 7 und 10 verarbeiten das Sumpfprodukt der zweiten Kolonne 6 mit. Es wird dazu mit dem zweiten Oligomerisat in einem zweiten Mischer 11 vermischt.

Im Regenerations-Betrieb dieser Ausführungsform II wird nun der im ersten Reaktor 4 befindliche, für die C₂-Oligomeriserung bestimmte Katalysator mit dem n-Buten C4 beaufschlagt, welches auch als Einsatzgemisch für die C₄-Oligomersierung im zweiten Reaktor 9 dient (Figur 4). Es wird also ein reaktives Spülmedium verwendet, welches gleichzeitig den C2-Katalystor spült und an diesem auch das als Spülmedium genutzte n-Buten oligomerisiert. Die dabei entstehende Reaktionswärme kann genutzt werden, um die Vorgabe-Temperatur für den ersten Reaktor 4 im Regenerations-Betrieb anzuheben. Bei der kombinierten Regeneration und C4-Oligomerisierung entsteht im ersten Reaktor 4 ein drittes Oligomerisat C4, C8, C12, C16, COKE, welches hinsichtlich seiner Komponenten dem zweiten Oligomerisat C4, C8, C12, C16 ähnelt, aber zusätzlich noch die gelösten Ablagerungen COKE enthält. Aufgrund der ähnlichen Zusammensetzung wird das verbrauchte Spülmedium (=drittes Oligomerisat C4, C8, C12, C16, COKE) über eine Kühlfalle 8 in den zweiten Mischer 11 gefahren, dort mit dem zweiten Oligomerisat C4, C8, C12, C16 vermischt und gemeinsam über die Kolonnen 7 und 10 aufgearbeitet. Die von dem für die C₂-Oligomeriserung bestimmten Katalysator abgelösten Ablagerungen COKE werden von der Kühlfalle 8 aufgefangen und gelangen nicht in den zweiten Reaktor 9.

Nach Abschluss der Regeneration (Figur 4) wird wieder in den Oligomerisierungs-Betrieb (Figur 3) umgeschaltet. Dabei verbleibt noch etwas n-Buten C4 im C₂-Strang, was dort Teil des Lösemittels SOLV wird.

### Beispiel 1: Oligomerisierung von Ethen in Isobutan sowie Regeneration des Katalysators mittels heißen Isobutan/Raffinat III - Gemisches

355 g eines heterogenen Katalysators basierend auf Nickel und Silica-Alumina (vgl. US 2581228) wurden in einen außen mit Öl temperierten, mit einem Thermoelement versehenen Rohrreaktor von 2 m Länge und 2.1 cm Innendurchmesser gefüllt. Anschließend wurden darüber in geradem Durchgang Gemische aus 2 bis 10 Gew.-% Ethen in Isobutan mit Gesamtmengenströmen von 0.96 bis 1.97 kg/h bei Vorgabe-Temperaturen von 30 bis 80 °C gefahren (WHSV = 2.7 bis 5.6 h⁻¹). Der Druck wurde auf 30*10⁵ Pa konstant gehalten. Nach einer Laufzeit von 692 h wurde das Ethen abgestellt und durch einen Gesamtstrom von 1.38 kg/h aus 80 Gew.-% Raffinat III und 20 Gew.-% Isobutan ersetzt. Die genaue Zusammensetzung dieses Spülmediums ist in Tabelle 1 dargestellt.

**Tabelle 1: Zusammensetzung Spülmedium**

| **1-Buten** | ***cis*-2-Buten** | ***trans*-2-Buten** | ***n*-Butan** | **Isobutan** | **Sonstige Stoffe** |
|---|---|---|---|---|---|
| [Gew.-%] | [Gew.-%] | [Gew.-%] | [Gew.-%] | [Gew.-%] | [Gew.-%] |
| 26 bis 29 | 11 bis 12 | 20 bis 22 | 17 | 22 | weniger als 0.5 |

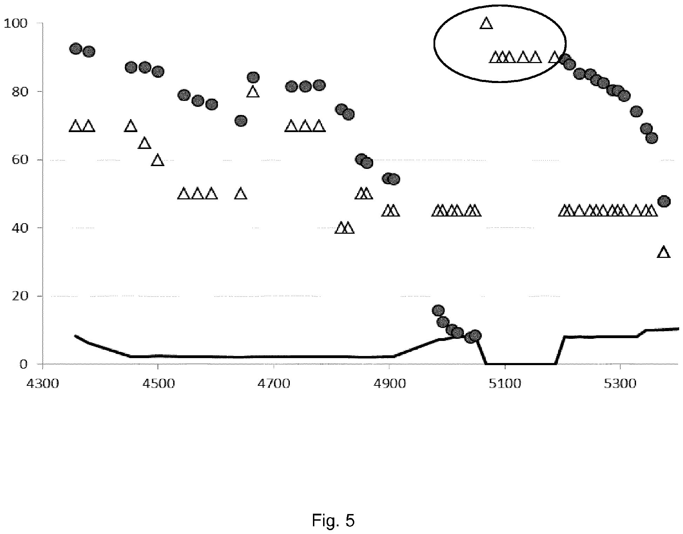

Mit diesem Spülmedium wurde der Katalysator für 119 h bei einer erhöhten Temperatur von kurzzeitig 100 °C und dann 90 °C "ausgekocht" (vgl. Figur 5, eingekreister Bereich). Anschließend wurde erneut auf 8% Ethen in 1.18 kg/h Isobutan umgestellt, womit ein Umsatz von 89% erreicht werden konnte. Die genauen Betriebsintervalle gehen aus Figur 5 hervor. Darin steht der Punkt • für den Ethen-Umsatz, das Dreieck Δ für die Vorgabe-Temperatur (Vorlauf-Temperatur des Öls) und die durchgezogene Linie - für den Anteil des Ethens in dem Reaktionsgemisch.

Somit konnte gezeigt werden, dass nach einer Desaktivierung des Katalysators über eine Laufzeit von 692 h, einhergehend mit einer Abnahme des Ethylen-Umsatzes von 93% auf 8% C₄- bis C₁₆-Oligomeren, durch Auskochen des Reaktors mit einem Gemisch aus Isobutan und Raffinat III bei 90 °C für 119 h ein Wiederanstieg des Umsatzes auf 89% und damit die Regeneration des Katalysators realisiert werden konnten.

### Beispiel 2: Oligomerisierung von Ethen in Isobutan sowie Regeneration des Katalysators mittels heißen Isobutans

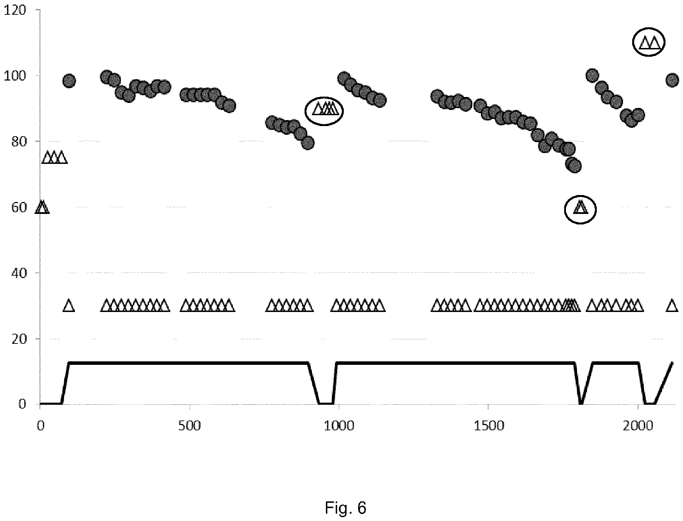

215 g eines heterogenen Katalysators basierend auf Nickel und Silica-Alumina (vgl. US 2581228) wurden in einen außen mit Öl temperierten, mit einem Thermoelement versehenen Rohrreaktor von 2 m Länge und 2.1 cm Innendurchmesser gefüllt. Anschließend wurde darüber in geradem Durchgang ein Gemisch aus durchschnittlich 15 Gew.-% Ethen und 85 Gew.-% Isobutan mit einem Gesamtmengenstrom von 1 kg/h bei einer Vorgabe-temperatur von 30 °C gefahren (WHSV = 4.7 h⁻¹). Der Druck wurde auf 30*10⁵ Pa konstant gehalten. In gewissen Abständen wurde die Ethen-Zufuhr unterbrochen und der Katalysator bei erhöhten Temperaturen mit einem Mengenstrom von 1 kg/h an purem Isobutan unterschiedlich lang "gespült". Dabei war es durch die Verwendung von 20 µm-Filtern möglich (ausgehend von insgesamt ca. 202 kg eingesetzten Ethens) insgesamt 17 g einer weißen pulvrigen Substanz zu isolieren, die sich als Polyethylen einer niedermolekularen Verteilung herausstellte. Die Details zu den Reaktions- und Regenerationsphasen sind in Figur 6 verbildlicht. In Figur 6 steht der Punkt • für den Ethen-Umsatz, das Dreieck Δ für die Vorgabe-Temperatur und die durchgezogene Linie - für den Anteil des Ethens in dem Reaktionsgemisch. Die Regenerationen sind eingekreist. Die Daten sind in Tabelle 2 zusammengefasst.

**Tabelle 2: Regenerationsprotokoll zu Beispiel 2**

| **Betriebsphasen** | **RegenerationsPhasen** | **Temperatur** | **Dauer [h]** | **Fortschritt der Desaktivierung (Umsatzverlust)** |
|---|---|---|---|---|
| 1. Intervall | | 30 °C | 840 h | 98% → 80% |
| | 1. Regeneration | 90 °C | 48 h | |
| 2. Intervall | | 30 °C | 624 h | 99% → 73% |
| | 2. Regeneration | 110 °C | 6 h | |
| 3. Intervall | | 30 °C | 168 h | 100% → 88% |
| | 3. Regeneration | 110 °C | 32 h | |

Hieraus lässt sich erkennen, dass bei einer Laufzeit von 840 Stunden der Ethylen-Umsatz zu C₄-bis C₁₆-Oligomeren von 98% auf 80% abfiel. Eine erste Regeneration mittels Isobutan bei 90 °C für 48 Stunden konnte den anschließenden Ethen-Umsatz wieder auf 99% anheben. Dieser sank während einer weiteren Laufzeit von 624 Stunden bei 30 °C wieder von 99% auf 73%. Ein zweiter Regenerationszyklus mittels Isobutan für 6 Stunden bei 110 °C konnte den anschließenden Ethen-Umsatz erneut auf 100% anheben. Nach einer weiteren Laufzeit von 168 Stunden sank der Ethen-Umsatz zu C₄- bis C₁₆-Oligomeren erneut von 100% auf 88%. Eine Regeneration mittels Isobutan bei 110 °C für 32 h ermöglichte einen erneuten Ethen-Umsatz von 99%.

### Beispiel 3: Gepulster Regenerations-Betrieb

In einem weiteren Versuch wurden 263 g eines heterogenen Katalysators basierend auf Nickel und Silica-Alumina in einen außen mit Öl temperierten, mit einem Thermoelement versehenen Rohrreaktor von 2 m Länge und 2.1 cm Innendurchmesser gefüllt. Anschließend wurde darüber in geradem Durchgang ein Gemisch aus durchschnittlich 13 Gew.-% Ethen und 87 Gew.-% Isobutan mit einem Gesamtmengenstrom von 1 kg/h bei einer Vorgabetemperatur von 35 °C bis 70 °C gefahren (WHSV = 3.8 h-1). Der Druck wurde auf 30*10⁵ Pa konstant gehalten.

In gewissen Abständen wurde die Ethen-Zufuhr unterbrochen und der Katalysator bei einer erhöhten Temperatur von 110 °C bis 120 °C mit einem Mengenstrom von 0,87 kg/h an purem Isobutan "gespült", wobei die Intervalle für den Oligomerisierungs-Betrieb und den Regenerations-Betrieb deutlich verkürzt wurden. Die Wechselfrequenz ergab sich aus der Überwachung des Ethen-Umsatzes (=(eingesetztes Ethen im Zulauf - unverbrauchtes Ethen im Austrag) / eingesetztes Ethen im Zulauf) im Oligomerisierungs-Betrieb: Sobald der kontinuierlich gemessene Umsatz des Ethens in der Oligomerisierung auf 97 % gefallen war, wurde eine kurze Regenerationsphase eingeschoben. Es wurde nach anfänglichen etwas längeren Regenerationsphasen festgestellt, dass unter diesen Reaktionsbedingungen ein Intervall von jeweils 12 Stunden scheinbar ausreicht, wobei diese 12 Stunden noch kein optimierter Wert sind. Um jedoch schleichende Veränderungen besser interpretieren zu können, wurde die Regenerationsdauer zur besseren Vergleichbarkeit konstant gehalten

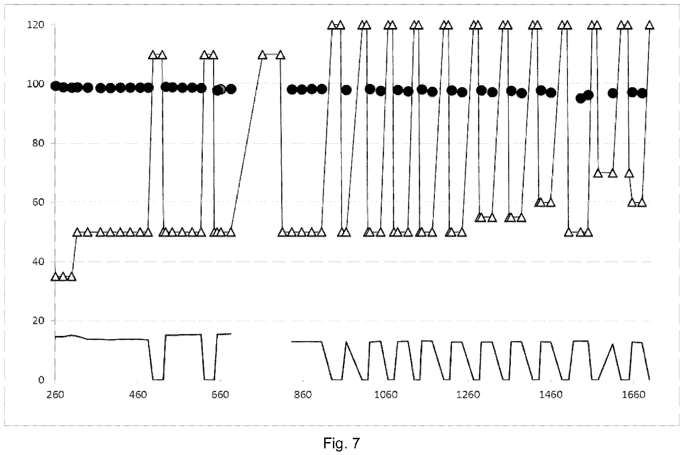

Die Betriebswechsel sind in Figur 7 dargestellt. Der Punkt • steht hierbei wieder für den Ethen-Umsatz, das Dreieck Δ für die Vorgabe-Temperatur und die durchgezogene Linie - für den Anteil des Ethens in dem Reaktionsgemisch.

Zu erkennen ist, dass der Oligomerisierungs-Betrieb auf diese Weise häufig durch eine impulsartige Regeneration unterbrochen wird. Zugleich wird deutlich, dass die Umsätze dadurch länger bei annähernd 100 % gehalten werden können und dass auch langfristig keine merklichen irreversiblen Aktivitätsverluste auftreten. Durch die Verwendung von 20 µm-Filtern war es wieder möglich (ausgehend von insgesamt ca. 150 kg eingesetzten Ethens) insgesamt etwa 2 g einer weißen pulvrigen Substanz zu isolieren, die sich als Polyethylen einer niedermolekularen Verteilung herausstellte. Die deutlich geringere Menge an Polyethylen im Vergleich zu der Menge in Beispiel 2 lässt erwarten, dass sich der Katalysator mit der gepulsten Fahrweise deutlich langlebiger verhält.

### Fazit

Der Ethen-Umsatz zu C₄- bis C₁₆-Oligomeren fällt ohne weiteres Eingreifen mit der Zeit linear immer weiter ab. Würde man den Katalysator nicht in gewissen Abständen wieder regenerieren, so würde der Umsatz deutlich unter 80% sinken. Es konnte gezeigt werden, dass es möglich ist ohne ein aufwändiges Ausbauen des Katalysators, sondern lediglich durch Abstellen der Ethen-Versorgung und Spülen mit dem verbleibenden heißen Lösemittel diesen wieder zu reaktivieren. Hierbei ist die gleichmäßig über den gesamten Reaktor eingestellte erhöhte Temperatur des Lösemittels zum erfolgreichen Herauslösen der Vergiftungskomponente, die für die Desaktivierung verantwortlich ist, entscheidend. Ein gepulster Regenerationsbetrieb ermöglicht eine bessere Langzeitstabilität des Katalysators.

### Bezugszeichenliste

- I: erste Ausführungsform
- II: zweite Ausführungsform
- 1: Mischer
- 2: Ethen-Rückführleitung
- 3: Lösemittel-Kreislauf
- 4: (erster) Reaktor (C2-Oli)
- 5: erste Kolonne
- 6: zweite Kolonne
- 7: dritte Kolonne
- 8: Kühlfalle
- 9: zweiter Reaktor (C4-Oli)
- 10: vierte Kolonne
- 11: zweiter Mischer
- C2: Ethen
- C2, C4, C6, C8, SOLV: Oligomerisat (aus C2-Oli im ersten Reaktor)
- C2, SOLV: Einsatzgemisch
- C2+: Olefine mit mehr als zwei Kohlenstoffatomen
- C4: Dimere des Ethens / n-Buten
- C4+: Olefine mit mehr als vier Kohlenstoffatomen
- C4, C8, C12, C16: zweites Oligomerisat (aus C4-Oli im zweiten Reaktor)
- C4, C8, C12, C16, COKE: drittes Oligomerisat (aus C4-Oli im ersten Reaktor)
- C6: Trimere des Ethens
- C8: Tetramere des Ethens / Dimere des n-Butens
- C12: Trimere des n-Butens
- C16: Tetramere des n-Butens
- COKE: Ablagerungen
- SOLV: Lösemittel

## Patentansprüche

1. Verfahren zur Oligomerisierung von Ethen an einem heterogenen Katalysator und zur Regeneration dieses Katalysators, mit den folgenden Merkmalen:
a) die Oligomerisierung erfolgt in einem Oligomerisierungs-Betrieb, in welchem Ethen, welches zumindest teilweise in einem flüssigen Lösemittel gelöst ist, mit dem heterogenen Katalysator kontaktiert wird;
b) die Regeneration erfolgt in einem Regenerations-Betrieb, in welchem der Katalysator in Abwesenheit von Ethen, Wasserstoff und Sauerstoff mit einem flüssigen Spülmedium gespült wird;
c) über die Zeit wird gewechselt zwischen dem Oligomerisierungs-Betrieb und dem Regenerations-Betrieb, sodass sich an einen zeitlich begrenzten Oligomerisierungs-Betrieb ein zeitlich begrenzter Regenerations-Betrieb anschließt, und an diesen wieder ein zeitlich begrenzter Oligomerisierungs-Betrieb;
d) der heterogene Katalysator befindet sich stets an demselben Ort, weswegen sowohl Oligomerisierungs-Betrieb als auch Regenerations-Betrieb an diesem Ort stattfinden;
e) der Ort, an dem sich der Katalysator befindet, wird mit Wärmenergie beaufschlagt, um ihm eine Vorgabe-Temperatur aufzuprägen, wobei die tatsächliche Temperatur am Katalysator zeitlich und örtlich begrenzt durchaus von der Vorgabe-Temperatur abweichen kann;
f) die Vorgabe-Temperatur im Regenerations-Betrieb ist höher als die Vorgabe-Temperatur im Oligomerisierungs-Betrieb.

2. Verfahren nach Anspruch 1, **dadurch gekennzeichnet, dass** sowohl das Lösemittel als auch das Spülmedium ausgewählt sind aus den Alkenen mit drei bis zwölf Kohlenstoffatomen oder aus den Alkanen mit drei bis zehn Kohlenstoffatomen oder aus Mischungen daraus, wobei die Cycloalkene und die Cycloalkane von dieser Aufzählung mit umfasst sind.

3. Verfahren nach Anspruch 2, **dadurch gekennzeichnet, dass** das Lösemittel und das Spülmedium identisch sind.

4. Verfahren nach Anspruch 3, **dadurch gekennzeichnet, dass** es sich bei dem Lösemittel und bei dem Spülmedium um eine der folgenden Substanzen handelt oder um ein Gemisch mehrerer dieser Substanzen: Propan, Isobutan, Pentan, Cyclopentan, Hexan, Cyclohexan, Heptan, Cycloheptan.

5. Verfahren nach Anspruch 1 oder nach einem der Ansprüche 2 bis 4, **dadurch gekennzeichnet, dass** die Vorgabe-Temperatur im Oligomerisierungs-Betrieb zwischen 20 °C und 130 °C liegt, und dass die Vorgabe-Temperatur im Regenerations-Betrieb zwischen 80 °C und 150 °C liegt; weithin unter der Maßgabe, dass die Vorgabe-Temperatur im Regenerationsbetrieb höher gewählt ist als die Vorgabe-Temperatur im Oligomerisierungs-Betrieb.

6. Verfahren nach Anspruch 5, **dadurch gekennzeichnet, dass** der Oligomerisierungs-Betrieb bei folgenden Bedingungen erfolgt:
| | |
|---|---|
| Druck: | 1*10⁵ Pa bis 50*10⁵ Pa |
| WHSV: | 2 h⁻¹ bis 50 h⁻¹ |
| Ethen-Anteil in Gesamtlösung: | 1 Gew.-% bis 50 Gew.-% |
und dass der Regenerations-Betrieb bei folgenden Bedingungen erfolgt:
| | |
|---|---|
| Druck: | 1*10⁵ Pa bis 50*10⁵ Pa |
| WHSV: | 2 h⁻¹ bis 50 h⁻¹ |
| Ethen-Anteil in Gesamtlösung: | 0 Gew.-% bis 1 Gew.-% |

7. Verfahren nach Anspruch 1 oder nach einem der Ansprüche 2 bis 6, **dadurch gekennzeichnet, dass** die Dauer eines Regenerations-Betriebs kürzer ist als die Dauer des vorhergehenden Oligomerisierungs-Betriebs, insbesondere, dass die Dauer des Regenerations-Betriebs weniger als 20 %, bevorzugt weniger als 10% und ganz besonders bevorzugt weniger als 5 % der Dauer des vorhergehenden Oligomerisierungs-Betriebs beträgt.

8. Verfahren nach Anspruch 1 oder nach einem der Ansprüche 2 bis 7, **dadurch gekennzeichnet, dass** der Ort, an dem sich der Katalysator befindet und an dem der Regenerations-Betrieb und der Oligomerisierungs-Betriebs erfolgen, ein Reaktor ist, welcher zum Zwecke der Beaufschlagung mit Wärmeenergie von einem Temperier-Medium durchflossen ist, und dass die Vorgabe-Temperatur durch die Vorlauf-Temperatur des Temperier-Mediums vorgegeben wird.

9. Verfahren nach Anspruch 1 oder nach einem der Ansprüche 2 bis 8, **dadurch gekennzeichnet, dass** im Regenerations-Betrieb das Spülmedium im Kreislauf geführt wird, und dass das im Kreislauf geführte Spülmedium außerhalb des Ortes, an dem sich der Katalysator befindet, mit Hilfe eines Trennapparats von zumindest einem Teil von im Spülmedium gelösten Komponenten gereinigt wird.

10. Verfahren nach Anspruch 9, **dadurch gekennzeichnet, dass** es sich bei dem Trennapparat um einen Filter oder um eine Membran oder um eine Kühlfalle handelt oder um eine Kombination mehrerer dieser Trennapparate.

11. Verfahren nach Anspruch 2 oder nach einem der Ansprüche 3 bis 8, wobei es sich bei dem Spülmedium um ein Alken mit drei bis zwölf Kohlenstoffatomen handelt oder um eine Mischung mehrerer solcher Alkene oder um eine Mischung eines oder mehrerer solcher Alkene mit mindestens einem Alkan mit drei bis sieben Kohlenstoffatomen, **dadurch gekennzeichnet, dass** im Regenerations-Betrieb das Spülmedium von dem Ort, an dem sich der Katalysator befindet, abgezogen und - optional nach einer Reinigung - auf einen zweiten heterogenen Katalysator geführt wird, der sich entfernt von dem zu regenerierenden Katalysator befindet, und dass mindestens ein im Spülmedium enthaltendes Alken an dem zweiten Katalysator einer chemischen Reaktion unterworfen wird, insbesondere einer zweiten Oligomerisierung, einer Isomerisierung oder einer Veretherung oder einer oxidativen Dehydrierung.

12. Verfahren nach Anspruch 2 oder nach einem der Ansprüche 3 bis 8, wobei es sich bei dem Spülmedium um ein Alkan mit drei bis sieben Kohlenstoffatomen handelt oder um eine Mischung mehrerer solcher Alkane, **dadurch gekennzeichnet, dass** im Regenerations-Betrieb das Spülmedium von dem Ort, an dem sich der Katalysator befindet, abgezogen und auf einen zweiten heterogenen Katalysator geführt wird, der sich entfernt von dem zu regenerierenden Katalysator befindet, und dass mindestens ein im Spülmedium enthaltendes Alkan an dem zweiten Katalysator einer chemischen Reaktion unterworfen wird, insbesondere einer Dehydrierung zu Alkoholen und/oder Säurederivaten.

13. Verfahren nach Anspruch 1 oder einem der Ansprüche 2 bis 12, **dadurch gekennzeichnet, dass** als heterogener Katalysator ein Festkörper eingesetzt wird, welcher mindestens zwei Komponenten enthält, wobei die erste Komponente mindestens ein aus Ni, Cr, Fe, Ti ausgewähltes Element umfasst, welches in metallischer und/oder oxidischer und/oder hydridischer Form vorliegt, und wobei die zweite Komponente mindestens ein aus Al₂O₃, SiO₂, TiO₂, ZrO₂ ausgewähltes Metalloxid umfasst.

14. Verfahren nach Anspruch 1 oder einem der Ansprüche 2 bis 13, **dadurch gekennzeichnet, dass** im Oligomerisierungs-Betrieb weniger als 5 Gew.-% des umgesetzten Ethens in Oligomere bzw. Polymere des Ethens mit sechzehn oder mehr als sechzehn Kohlenstoffatomen umgesetzt werden.

15. Verfahren nach Anspruch 1 oder einem der Ansprüche 2 bis 14, **dadurch gekennzeichnet, dass** der Umsatz des Ethens im Oligomerisierungs-Betrieb kontinuierlich bestimmt wird, und dass dann von dem Oligomerisierungs-Betrieb in Regenerations-Betrieb gewechselt wird, wenn der Umsatz des Ethens auf einen Wert zwischen 95 % und 100 % gefallen ist.

## Claims

1. Process for oligomerizing ethene over a heterogeneous catalyst and for regenerating said catalyst, having the following features:
a) the oligomerization is effected in an oligomerization operation in which ethene at least partly dissolved in a liquid solvent is contacted with the heterogeneous catalyst;
b) the regeneration is effected in a regeneration operation in which the catalyst in the absence of ethene, hydrogen and oxygen is purged with a liquid purge medium;
c) oligomerization operation and regeneration operation alternate over time, such that a time-limited oligomerization operation is followed by a time-limited regeneration operation, and the latter in turn by a time-limited oligomerization operation;
d) the heterogeneous catalyst is always at the same location, which is the reason why both oligomerization operation and regeneration operation take place at this location;
e) the location of the catalyst is supplied with thermal energy in order to impose a set temperature thereon, it being entirely possible for the actual temperature at the catalyst to deviate from the set temperature in a time- and space-limited manner;
f) the set temperature in regeneration operation is higher than the set temperature in oligomerization operation.

2. Process according to Claim 1, **characterized in that** both the solvent and the purge medium are selected from the alkenes having three to twelve carbon atoms or from the alkenes having three to ten carbon atoms or from mixtures thereof, this enumeration including the cycloalkenes and the cycloalkanes.

3. Process according to Claim 2, **characterized in that** the solvent and the purge medium are identical.

4. Process according to Claim 3, **characterized in that** the solvent and the purge medium are one of the following substances or a mixture of two or more of these substances: propane, isobutane, pentane, cyclopentane, hexane, cyclohexane, heptane, cycloheptane.

5. Process according to Claim 1 or according to any of Claims 2 to 4, **characterized in that** the set temperature in oligomerization operation is between 20°C and 130°C, and **in that** the set temperature in regeneration operation is between 80°C and 150°C; additionally with the proviso that the set temperature chosen in regeneration operation is higher than the set temperature in oligomerization operation.

6. Process according to Claim 5, **characterized in that** the oligomerization operation is effected under the following conditions:
| | |
|---|---|
| Pressure: | 1*10⁵ Pa to 50*10⁵ Pa |
| WHSV: | 2 h⁻¹ to 50 h⁻¹ |
Ethene content in overall solution:1% by weight to 50% by weight
and **in that** the regeneration operation is effected under the following conditions:
| | |
|---|---|
| Pressure: | 1*10⁵ Pa to 50*10⁵ Pa |
| WHSV: | 2 h⁻¹ to 50 h⁻¹ |
Ethene content in overall solution:0% by weight to 1% by weight

7. Process according to Claim 1 or according to any of Claims 2 to 6, **characterized in that** the duration of a regeneration operation is shorter than the duration of the preceding oligomerization operation, especially **in that** the duration of the regeneration operation is less than 20%, preferably less than 10% and most preferably less than 5% of the duration of the preceding oligomerization operation.

8. Process according to Claim 1 or according to any of Claims 2 to 7, **characterized in that** the location of the catalyst where the regeneration operation and the oligomerization operation are effected is a reactor through which a temperature control medium flows for the purpose of supplying thermal energy, and **in that** the set temperature is set by the feed temperature of the temperature control medium.

9. Process according to Claim 1 or according to any of Claims 2 to 8, **characterized in that** the purge medium is circulated in regeneration operation, and **in that** the purge medium circulated, away from the location of the catalyst, is purified with the aid of a separation apparatus to free it of at least some components dissolved in the purge medium.

10. Process according to Claim 9, **characterized in that** the separation apparatus is a filter or a membrane or a cold trap, or a combination of two or more of these separation apparatuses.

11. Process according to Claim 2 or according to any of Claims 3 to 8, wherein the purge medium is an alkene having three to twelve carbon atoms or a mixture of two or more such alkenes or a mixture of one or more such alkenes with at least one alkane having three to seven carbon atoms, **characterized in that** the purge medium in regeneration operation is drawn off from the location of the catalyst and - optionally after purification - conducted to a second heterogeneous catalyst remote from the catalyst to be regenerated, and **in that** at least one alkene present in the purge medium is subjected to a chemical reaction over the second catalyst, especially a second oligomerization, an isomerization or an etherification or an oxidative dehydrogenation.

12. Process according to Claim 2 or according to any of Claims 3 to 8, wherein the purge medium is an alkane having three to seven carbon atoms or a mixture of two or more such alkanes, **characterized in that** the purge medium in regeneration operation is drawn off from the location of the catalyst and conducted to a second heterogeneous catalyst remote from the catalyst to be regenerated, and **in that** at least one alkane present in the purge medium is subjected to a chemical reaction over the second catalyst, especially a dehydrogenation to give alcohols and/or acid derivatives.

13. Process according to Claim 1 or any of Claims 2 to 12, **characterized in that** the heterogeneous catalyst used is a solid containing at least two components, the first component containing at least one element selected from Ni, Cr, Fe, Ti and being in metallic and/or oxidic and/or hydridic form, and the second component comprising at least one metal oxide selected from Al₂O₃, SiO₂, TiO₂, ZrO₂.

14. Process according to Claim 1 or any of Claims 2 to 13, **characterized in that** less than 5% by weight of the ethene converted in oligomerization operation is converted to oligomers or polymers of ethene having sixteen or more than sixteen carbon atoms.

15. Process according to Claim 1 or any of Claims 2 to 14, **characterized in that** the conversion of ethene is determined continuously in oligomerization operation, and **in that** there is then a changeover from oligomerization operation to regeneration operation when the conversion of ethene has dropped to a value between 95% and 100%.

## Revendications

1. Procédé d'oligomérisation d'éthène sur un catalyseur hétérogène et de régénération de ce catalyseur, présentant les caractéristiques suivantes :
a) l'oligomérisation a lieu dans un mode d'oligomérisation, dans lequel l'éthène, qui est au moins partiellement dissous dans un solvant liquide, est mis en contact avec le catalyseur hétérogène ;
b) la régénération a lieu dans un mode de régénération, dans lequel le catalyseur est rincé avec un milieu de rinçage liquide en l'absence d'éthène, d'hydrogène et d'oxygène ;
c) on alterne dans le temps entre le mode d'oligomérisation et le mode de régénération, de sorte qu'un mode d'oligomérisation limité dans le temps soit suivi par un mode de régénération limité dans le temps, qui est de nouveau suivi par un mode d'oligomérisation limité dans le temps ;
d) le catalyseur hétérogène se trouve toujours au même emplacement, raison pour laquelle aussi bien le mode d'oligomérisation que le mode de régénération ont lieu à cet emplacement ;
e) l'emplacement où se trouve le catalyseur est chargé avec une énergie thermique afin de le porter à une température spécifiée, la température réelle sur le catalyseur pouvant dévier dans le temps et dans l'espace de la température spécifiée d'une manière limitée ;
f) la température spécifiée dans le mode de régénération est supérieure à la température spécifiée dans le mode d'oligomérisation.

2. Procédé selon la revendication 1, **caractérisé en ce qu'**aussi bien le solvant que le milieu de rinçage sont choisis parmi les alcènes contenant trois à douze atomes de carbone ou les alcanes contenant trois à dix atomes de carbone ou leurs mélanges, les cycloalcènes et les cycloalcanes étant compris dans cette liste.

3. Procédé selon la revendication 2, **caractérisé en ce que** le solvant et le milieu de rinçage sont identiques.

4. Procédé selon la revendication 3, **caractérisé en ce que** le solvant et le milieu de rinçage sont une des substances suivantes ou un mélange de plusieurs de ces substances : propane, isobutane, pentane, cyclopentane, hexane, cyclohexane, heptane, cycloheptane.

5. Procédé selon la revendication 1 ou selon l'une quelconque des revendications 2 à 4, **caractérisé en ce que** la température spécifiée dans le mode d'oligomérisation est comprise entre 20 °C et 130 °C, et **en ce que** la température spécifiée dans le mode de régénération est comprise entre 80 °C et 150 °C ; en outre à condition que la température spécifiée dans le mode de régénération soit choisie supérieure à la température spécifiée dans le mode d'oligomérisation.

6. Procédé selon la revendication 5, **caractérisé en ce que** le mode d'oligomérisation a lieu dans les conditions suivantes :
| | |
|---|---|
| pression : | 1*10⁵ Pa à 50*10⁵ Pa |
| WHSV : | 2 h⁻¹ à 50 h⁻¹ |
proportion d'éthylène dans la solution totale : 1 % en poids à 50 % en poids,
et **en ce que** le mode de régénération a lieu dans les conditions suivantes :
| | |
|---|---|
| pression : | 1*10⁵ Pa à 50*10⁵ Pa |
| WHSV : | 2 h⁻¹ à 50 h⁻¹ |
proportion d'éthylène dans la solution totale : 0 % en poids à 1 % en poids.

7. Procédé selon la revendication 1 ou selon l'une quelconque des revendications 2 à 6, **caractérisé en ce que** la durée d'un mode de régénération est plus courte que la durée du mode d'oligomérisation précédent, notamment **en ce que** la durée du mode de régénération est de moins de 20 %, de préférence de moins de 10 % et de manière particulièrement préférée de moins de 5 % de la durée du mode d'oligomérisation précédent.

8. Procédé selon la revendication 1 ou selon l'une quelconque des revendications 2 à 7, **caractérisé en ce que** l'emplacement où se trouve le catalyseur et où le mode de régénération et le mode d'oligomérisation ont lieu est un réacteur, qui est traversé par un milieu de conditionnement dans le but du chargement avec une énergie thermique, et **en ce que** la température prédéfinie est prédéfinie par la température du flux du milieu de conditionnement.

9. Procédé selon la revendication 1 ou selon l'une quelconque des revendications 2 à 8, **caractérisé en ce que**, dans le mode de régénération, le milieu de rinçage est mis en circulation dans un circuit, et **en ce que** le milieu de rinçage mis en circulation dans un circuit est débarrassé d'au moins une partie des composants dissous dans le milieu de rinçage en dehors de l'emplacement où se trouve le catalyseur à l'aide d'un appareil de séparation.

10. Procédé selon la revendication 9, **caractérisé en ce que** l'appareil de séparation est un filtre ou une membrane ou un piège froid ou une combinaison de plusieurs de ces appareils de séparation.

11. Procédé selon la revendication 2 ou selon l'une quelconque des revendications 3 à 8, le milieu de rinçage étant un alcène contenant trois à douze atomes de carbone ou un mélange de plusieurs tels alcènes ou un mélange d'un ou de plusieurs tels alcènes avec au moins un alcane contenant trois à sept atomes de carbone, **caractérisé en ce que**, dans le mode de régénération, le milieu de rinçage est soutiré de l'emplacement où se trouve le catalyseur et, éventuellement après une purification, conduit sur un deuxième catalyseur hétérogène, qui est éloigné du catalyseur à régénérer, et **en ce qu'**au moins un alcène contenu dans le milieu de rinçage est soumis sur le deuxième catalyseur à une réaction chimique, notamment à une deuxième oligomérisation, une isomérisation ou une éthérification ou une déshydrogénation oxydative.

12. Procédé selon la revendication 2 ou selon l'une quelconque des revendications 3 à 8, le milieu de rinçage étant un alcane contenant trois à sept atomes de carbone ou un mélange de plusieurs tels alcanes, **caractérisé en ce que**, dans le mode de régénération, le milieu de rinçage est soutiré de l'emplacement où se trouve le catalyseur et conduit sur un deuxième catalyseur hétérogène, qui est éloigné du catalyseur à régénérer, et **en ce qu'**au moins un alcane contenu dans le milieu de rinçage est soumis sur le deuxième catalyseur à une réaction chimique, notamment à une déshydrogénation en alcools et/ou dérivés d'acides.

13. Procédé selon la revendication 1 ou l'une quelconque des revendications 2 à 12, **caractérisé en ce qu'**un corps solide est utilisé en tant que catalyseur hétérogène, qui contient au moins deux composants, le premier composant comprenant au moins un élément choisi parmi Ni, Cr, Fe, Ti, qui se présente sous forme métallique et/ou oxydique et/ou hydrurique, et le deuxième composant comprenant au moins un oxyde métallique choisi parmi Al₂O₃, SiO₂, TiO₂, ZrO₂.

14. Procédé selon la revendication 1 ou l'une quelconque des revendications 2 à 13, **caractérisé en ce que**, dans le mode d'oligomérisation, moins de 5 % en poids de l'éthène mis en réaction est transformé en oligomères ou polymères de l'éthène contenant seize ou plus de seize atomes de carbone.

15. Procédé selon la revendication 1 ou l'une quelconque des revendications 2 à 14, **caractérisé en ce que** la conversion de l'éthène dans le mode d'oligomérisation est déterminée en continu, et **en ce que** l'on passe du mode d'oligomérisation au mode de régénération lorsque la conversion de l'éthène a chuté à une valeur comprise entre 95 % et 100 %.
